# EUROPÄISCHE PATENTSCHRIFT

(11) **EP 2 099 773 B1**
(45) Veröffentlichungstag und Bekanntmachung des Hinweises auf die Patenterteilung: **05.07.2017**
(21) Anmeldenummer: 07845264.6
(22) Anmeldetag: 23.11.2007
(51) Int. Cl.: C07D 241/08, C07C 229/16

(54) **NEUES VERFAHREN ZUR HERSTELLUNG VON 4,4'-(1-METHYL-1,2-ETHANDIYL)-BIS-(2,6-PIPERAZINDION)**
NOVEL METHOD FOR PRODUCING 4,4'-(1-METHYL-1,2-ETHANEDIYL)-BIS-(2,6-PIPERAZINEDIONE)
PROCÉDÉ DE FABRICATION DE 4,4'-(1-MÉTHYL-1,2-ÉTHANDIYL)-BIS-(2,6-PIPÉRAZINDIONE)

(30) Priorität: 24.11.2006 AT 19582006
(43) Veröffentlichungstag der Anmeldung: 16.09.2009
(73) Patentinhaber: Cyathus Exquirere Pharmaforschungs Gmbh, 1010 Wien (AT)
(72) Erfinder: KOCH, Andreas, 4020 Linz (AT); NEUFELLNER, Erwin, 4020 Linz (AT)
(74) Vertreter: Schober, Elisabeth
(86) Internationale Anmeldenummer: PCT/AT2007/000529
(87) Internationale Veröffentlichungsnummer: WO 2008/061270

(56) Entgegenhaltungen:
- EP-A- 0 330 381
- GB-A- 961 065
- US-A- 2 428 353
- US-A- 3 196 153
- US-A- 3 941 790
- WITIAK D T ET AL: "Study of trans-cyclopropylbis (diketopiperazine) and chelating agents related to ICRF 159. Cytotoxicity, mutagenicity, and effects on scheduled and unscheduled DNA synthesis." JOURNAL OF MEDICINAL CHEMISTRY MAY 1977, Bd. 20, Nr. 5, Mai 1977 (1977-05), Seiten 630-635, XP002469821 ISSN: 0022-2623
- HERMAN E H ET AL: "COMPARISON OF THE PROTECTIVE EFFECT OF ICRF-187 AND STRUCTURALLY RELATED ANALOGUES AGAINST ACUTE DAUNORUBICIN TOXICITY IN SYRIAN GOLDEN HAMSTERS" RESEARCH COMMUNICATIONS IN CHEMICAL PATHOLOGY AND PHARMACOLOGY, PJD PUBLICATIONS LTD., WESTBURY, NY, US, Bd. 48, Nr. 1, 1. April 1985 (1985-04-01), Seiten 39-55, XP000647002 ISSN: 0034-5164
- DWYER F P ET AL: "The Preparation of 1,2-Propylenediaminetetraacetic Acid and its Resolution through the Cobalt(II) Complex", JOURNAL OF THE AMERICAN CHEMICAL SOCIETY, ACS PUBLICATIONS, US, vol. 81, 1 January 1959 (1959-01-01), pages 2955-2957, XP002470465, ISSN: 0002-7863, DOI: 10.1021/JA01521A010

## Beschreibung

### Neues Verfahren zur Herstellung von 4,4'-(1-Methyl-1,2-ethandiyl)-bis-(2,6-piperazindion)

Die Erfindung bezieht sich auf ein neues Verfahren zur Herstellung von 4,4'-(1-Methyl-1,2-ethandiyl)-bis-(2,6-pipera-zindion). Insbesondere betrifft die Erfindung ein neues Verfahren zur Herstellung von 4,4'-(1-Methyl-1,2-ethandiyl)-bis-(2,6-piperazindion) in verbesserter Qualität und Ausbeute.

### 4,4'-(1-Methyl-1,2-ethandiyl)-bis-(2,6-piperazindion) besitzt die allgemeine Formel (I)

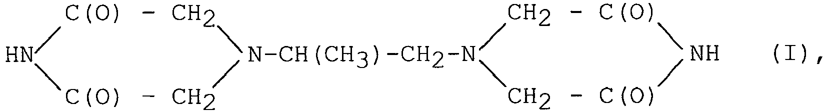

Die Verbindung der Formel (I) kann in Form zweier Enantiomere als (S)-(+)-4,4'-(1-Methyl-1,2-ethandiyl)-bis-(2,6-pipera-zindion), auch als Dexrazoxan bezeichnet, sowie als (R)-(-)-4,4'-(1-Methyl-1,2-ethandiyl)-bis-(2,6-piperazindion), auch als Levorazoxan bezeichnet, sowie in Form eines Racemats, (S,R)-4,4'-(1-Methyl-1,2-ethandiyl)-bis(2,6-piperazindion), auch als Razoxan bezeichnet, vorliegen. Im Zusammenhang mit dieser Erfindung wird unter "Verbindung der Formel (I)" bzw. "4,4'-(1-Methyl-1,2-ethandiyl)-bis-(2,6-piperazindion)" sowohl das S-Enantiomer, das R-Enantiomer als auch das Racemat verstanden.

Unabhängig von ihrer Stereochemie besitzt die Verbindung der Formel (I) Antitumorwirkung. Besondere Bedeutung hat in der Vergangenheit das S-Enantiomer der Verbindung der Formel (I), das Dexrazoxan, erlangt, von welchem bekannt ist, dass es gegen Tumore und andere Krebsformen wirksam und auch als Synergist in Kombination mit anderen Antikrebsmitteln nützlich ist. Insbesondere wurde festgestellt, dass Dexrazoxan im Hinblick auf Sarkom, Lymphosarkom und Leukämie Aktivität zeigt und besonders wirksam ist, wenn es in einem Regime in Verbindung mit Adriamycin verwendet wird.

In der Technik sind seit langem verschiedene Herstellungsverfahren für die Verbindung der Formel (I) bekannt. So werden in den US-Patenten Nr. 3.941.790 und Nr. 4.275.063, Creighton, drei Verfahren zur Herstellung von Bisdiketopiperazinen, wozu auch die Verbindungen der Formel (I) zählen, beschrieben. Im ersten Verfahren wird (S)-1,2-Diaminopropan mit Chloressigsäure umgesetzt, um (S)-1,2-Diaminopropantetraessigsäure auszubilden. Die Tetraessigsäure wird anschließend mit Formamid unter Stickstoff bei erhöhter Temperatur zur entsprechenden Verbindung der Formel (I) umgesetzt. Im zweiten Verfahren wird die Tetraessigsäure wie vorstehend beschrieben hergestellt, durch Umsetzung mit Ammoniak in das entsprechende Tetraessigsäureamid übergeführt und dieses anschließend durch Erhitzen in Polyphosphorsäure oder Phenol cyclisiert. Dieses Verfahren soll besonders vorteilhaft sein, wenn die Tetraessigsäure beim Erhitzen eine Tendenz zur Decarboxylierung zeigt. Als drittes Verfahren wird die Umsetzung eines Tetranitrils mit Natriumamid in Formamid und das anschließende Behandeln des entstandenen Produkts mit Chlorwasserstoff in Methanol erwähnt. Nach Creighton besitzt dieses alternative Verfahren den Vorteil, ein Niedrigtemperaturverfahren zu sein. Bei allen diesen Verfahren handelt es sich um stereoselektive Verfahren, dh die eingesetzten Zwischenverbindungen in Form der Tetraessigsäure, des Tetraamids oder des Tetranitrils sollten daher bereits in der für die Verbindung der Formel (I) gewünschten stereochemischen Konfiguration vorliegen.

Die in den vorstehenden Verfahren eingesetzten Zwischenverbindungen, wie die Tetraessigsäure, können auf verschiedenen Wegen hergestellt werden. Neben den bereits vorstehend angeführten Herstellungsverfahren wird beispielsweise im Britischen Patent Nr. 978.724, J.R. Geigy AG, ein Verfahren zur Ausbildung der Tetraessigsäure beschrieben, worin Diamine mit Formaldehyd und Wasserstoffcyanid umgesetzt werden, um ein Tetranitril auszubilden, welches verseift wird. Im US-Patent Nr. 2.461.519, Bersworth et al., wird ein Verfahren zur Herstellung von 1,2-Diaminopropantetracarbonsäure durch Umsetzen von 1,2-Diaminopropan mit Formaldehyd und Natriumcyanid bei alkalischem pH-Wert gelehrt.

Ein Hauptproblem bei der Herstellung der Verbindung der Formel (I) stellt im allgemeinen die Reinigung der Zwischenverbindungen dar, welche aufwendig und im kommerziellen Maßstab schwierig zu erzielen ist. So werden bei zahlreichen Verfahren die Zwischenverbindungen, wie beispielsweise die Tetraessigsäure, gemeinsam mit großen Mengen an Alkalimetallsalzen als Nebenprodukt erhalten, welche vor der Cyclisierung zur Verbindung der Formel (I) abgetrennt werden müssen.

Diese Probleme der vorstehend genannten Herstellungsverfahren beruhen insbesondere darauf, dass die eingesetzte Tetraessigsäure ebenso wie die Tetraamide, die Tetranitrile und die Verbindung der Formel (I) selbst sehr polare hydrophile Substanzen sind und mit den starken Basen, wie sie in den Herstellungsverfahren benötigt werden, Salze ausbilden. Dies führt in der Folge immer zu Schwierigkeiten bei der erforderlichen Abtrennung der nicht umgesetzten Vorläuferverbindungenund der entstandenen Nebenprodukte.

Die sich bei und durch die Reinigung der Verläuferverbindung in den bekannten Herstellungsverfahren ergebenden Probleme sind detailliert in der internationalen Patentanmeldung Nr. 93/08172, P.L. MacDonald, beschrieben. Zur Lösung dieser Probleme wird daher ein Verfahren zur Herstellung der Verbindung der Formel (I), konkret von Deraxozan, vorgeschlagen, bei welchem dieses in hohen Ausbeuten erhalten werden soll, ohne dass vor der Cyclisierung zum Dexrazoxan eine Reinigung der Tetraessigsäure-Zwischenverbindung erfolgt. Das Dexrazoxan wird durch dieses Verfahren jedoch gemeinsam mit größeren Mengen an Salz-Nebenprodukten erhalten, was zu Schwierigkeiten bei der Gewinnung von salzfreiem Dexrazoxan führt.

Neben Verfahren zur Herstellung von Verbindungen der Formel (I) oder analogen Verbindungen hievon, worin Tetraessigsäure, Tetraamid oder Tetranitril als Zwischenprodukt eingesetzt wird, ist in der Literatur auch ein Verfahren zur Herstellung von cis- und trans-Cyclopropyl-bis-2,6-(piperazindion), zweier zur Verbindung der Formel (I) analoger Verbindungen, beschrieben, welches über den entsprechenden Tetraessigsäuremethylester als Vorläuferverbindung verläuft. D.T. Witiak et al, Journal of Medicinal Chemistry, Bd. 20, Nr. 5, Seiten 630-635 (1977), und Journal of Medicinal Chemistry, Bd. 21, Nr. 12, Seiten 1194-1197 (1978), beschreiben zur Herstellung der trans-Verbindung die Cyclisierung des entsprechenden Tetraessigsäuremethylesters in Form des Hydrochlorids mit einem Überschuss von Ammoniak und Natriummethoxid in Methanol. Die Ausbeute an der gewünschten trans-Verbindung ist gering und beträgt lediglich 27% vor der Reinigung. Die Anwendung dieses Verfahren zur Herstellung der entsprechenden cis-Verbindung war laut den Autoren nicht erfolgreich: Zur Herstellung der cis-Verbindung wird der Tetraessigsäuremethylester mit Natriumhydrid und Formamid in DME cyclisiert. Die Ausbeute an der trans-Verbindung wird mit 36,5% angegeben.

Der Tetraessigsäuremethylester wird von Witiak et al. ausschließlich zur Herstellung der vorstehend genannten Verbindungen vorgeschlagen. Ein Hinweis, Tetraessigsäuremethylester-Verbindungen als Vorläuferverbindungen zur Herstellung von analogen Verbindungen einzusetzen, findet sich darin nicht. Die Probleme bei der Herstellung von cis- und trans-Isomer der gewünschten Verbindung legen vielmehr nahe, dass der Einsatz derartiger Verbindungen als Vorläuferverbindung nicht ohne weiteres möglich ist.

Ziel der vorliegenden Erfindung ist es, ein Verfahren zur Herstellung der Verbindung der Formel (I) bereitzustellen, welches die Herstellung dieser Verbindung in guter Ausbeute auch im kommerziellen Maßstab ermöglicht und die Probleme der technikbekannten Verfahren überwindet.

Dieses Ziel wird durch das erfindungsgemäße Verfahren erreicht, welches den Schritt des Cyclisierens eines 1,2-Diaminopropan-N,N,N',N'-tetraessigsäurealkylesters (nachstehend als "Tetraessigsäurealkylester" bezeichnet) mit Ammoniak in Formamid umfasst, wobei "Alkyl" hierin bevorzugt für (C₁-C₃)Alkyl steht, und "C₃-Alkyl" n-Propyl, Isopropyl als auch Cyclopropyl umfaßt.

Das erfindungsgemäße Verfahren basiert auf der Feststellung der überraschenden Eigenschaften der Alkylester der Tetraessigsäure, wie einer im Vergleich zu den bekannten Zwischenverbindungen verringerten Polarität und Hydrophilität, was zur Bereitstellung eines verbesserten Verfahrens zur Herstellung der Verbindung der Formel (I) verwendet wird. Darüber hinaus kann aufgrund der höheren Reaktivität dieser Ester der Ringschluss zur Verbindung der Formel (I) unter einfacheren Bedingungen, was die Anzahl der erforderlichen Reaktionsschritte und -maßnahmen als auch die dazu erforderlichen Reaktionsbedingungen betrifft, erzielt werden.

Weitere Vorteile des erfindungsgemäßen Verfahrens sind der Einsatz von Ammoniak und Formamid, zwei gebräuchlichen Chemikalien, wobei Formamid im erfindungsgemäßen Verfahren auch als Lösungsmittel eingesetzt wird. Der im Zuge der Cyclisierung entstehende Methanol kann durch einfache Destillation aus dem Reaktionsgemisch entfernt werden. Weitere Details zum erfindungsgemäßen Verfahren und seiner bevorzugten Ausführungsform sind auch den nachstehenden Beispielen zu entnehmen.

Im erfindungsgemäßen Verfahren werden der Formel (II)

(ROOCCH₂)₂N-CHCH₃-CH₂-N(CH₂COOR)₂ (II)

verwendet, worin R für Alkyl steht. Bevorzugt handelt es sich bei R um (C₁-C₃)Alkyl, wie Methyl, Ethyl oder Propyl.

Die Verbindungen der Formel (II), welche wertvolle Vorläuferverbindungen für die Verbindungen der Formel (I) sind, sind mit Ausnahme des Tetraessigsäuremethylesters, welcher von E.H. Herman et al. in Research Communications in Chemical Pathology and Pharmacology, Bd. 48, Nr. 1, Seiten 39-55 (1985,) beschrieben ist, neue Verbindungen. Die Tetraessigsäurealkylester können unter Verwendung technikbekannter Verfahren hergestellt werden, wie sie auch in den nachstehenden Beispielen beschrieben sind.

Das Verfahren zur Herstellung der Tetraessigsäurealkylester der vorliegenden Erfindung besteht in der Umsetzung eines Diamins der Formel (III)

H₂N-CHCH₃-CH₂-NH₂ (III)

mit Chloressigsäure und der darauffolgenden Behandlung mit Alkylalkohol zur Ausbildung des Tetraessigsäurealkylesters der Formel (II):

(ROOCCH₂)₂N-CHCH₃-CH₂-N(CH₂COOR)₂ (II),

worin R für Alkyl, vorzugsweise (C₁-C₃)Alkyl, wie Methyl, Ethyl oder Propyl, steht.

Die derart erhaltenen Tetraessigsäurealkylester werden in einem weiteren Schritt der Cyclisierung in Gegenwart von Ammoniak und Formamid unterzogen, um die Verbindung der Formel (I) zu erhalten.

Die Tetraessigsäurealkylester der Formel (II) können vor der Cyclisierung zur Verbindung der Formel (I) gewünschtenfalls einer Reinigung unterworfen werden, indem diese beispielsweise einer Verteilung zwischen einem mit Wasser nicht mischbaren Lösungsmittel und Wasser zur Abtrennung der gebildeten Alkalimetallsalze unterworfen werden. Als mit Wasser nicht mischbare Lösungsmittel kommen insbesondere Ethylacetat und Isopropylacetat zur Anwendung.

Die Tetraessigsäurealkylester der Formel (II) können jedoch auch ohne vorherige Reinigung zur Verbindung der Formel (I) cyclisiert werden. Diese Verfahrensvariante stellt eine besonders bevorzugte Ausführungsform der Verfahrens zur Herstellung der Verbindung der Formel (I) dar.

Auch in der besonderen Ausführungsform des erfindungsgemäße Verfahrens werden im Vergleich zu bekannten Verfahren höhere Ausbeuten an der Verbindung der Formel (I) und eine ausreichende Reinigung der Verbindung der Formel (I) erzielt. Zusätzlich wird eine weitere Reinigung und eine Isolierung der Tetraessigsäurealkylester, welche als Vorläuferverbindung eingesetzt werden, nicht erforderlich.

Im erfindungsgemäßen Verfahren wird ebenso wie in dessen bevorzugter Ausführungsform eine mögliche Zerstörung der Verbindung der Formel (I) durch Hydrolyse während des Verfahrens minimiert. Die Abtrennung von ionischen Materialien (wie der Alkalimetallsalze) kann vollständig und einfach durch Verteilung der Tetraessigsäurealkylester der Formel (II) zwischen einem mit Wasser nicht mischbaren Lösungsmittel, z.B. Ethylacetat, Isopropylacetat, und Wasser durchgeführt werden.

Das erfindungsgemäße Verfahren sowie die bevorzugte Ausführungsform desselben sind stereoselektive Verfahren, dh, dass die Vorläuferverbindung in der für die Verbindung der Formel (I) gewünschten Konfiguration vorliegen muss.

Weitere Aspekte der vorliegenden Erfindung sind aus den folgenden Beispielen entnehmbar, welche zu Zwecken der Veranschaulichung und nicht zur Einschränkung der Erfindung angeführt sind. Es wird dem Fachmann klar sein, dass die in den folgenden Beispielen beschriebenen Verfahrensdetails innerhalb des Rahmens der vorliegenden Erfindung modifiziert werden können. So können nach dem für das S-Enantiomer, Dexrazoxan, beschriebenen Verfahren des Beispiels 5 auch das R-Enantiomer, Levorazoxan, sowie das Racemat hergestellt werden. Soweit nicht anders angeführt oder sich aus dem Zusammenhang ergebend, beziehen sich die Prozentsätze auf das Gewicht.

### Beispiele

### Beispiel 1

### (Vergleichsbeispiel)

### Herstellung von (S)-(+)-1,2-Diaminopropan-N,N,N',N'-tetraessigsäure

In vorgelegte 780,0 g deionisiertes Wasser werden bei RT 150,0 g (1,02 Mol) (S)-(-)-1,2-Diaminopropandihydrochlorid eingebracht, 578,4 g (6,12 Mol) Chloressigsäure zugesetzt und dieser Lösung unter Kühlung (bei 15°C) 1785,0 g (14,28 Mol) Natronlauge 32 Gew.-% über 45 Min zudosiert. Nach erfolgter Zugabe wird das Reaktionsgemisch auf 40°C erwärmt, wobei ab 40°C die Reaktion exotherm und die Temperatur unter Kühlung bei 40-45°C gehalten wird. Nach Abklingen der Exothermie wird 90 H bei 40-45°C gerührt. Die alkalische, farblose und klare Flüssigkeit wird unter Vakuum bei 70°C Badtemperatur um ca das 2,5 fache eingeengt. Der ölige Kristallbrei wird mit 1,2 1 Methanol versetzt, auf 20°C abgekühlt, die Salze werden abfiltriert und der Rückstand im Filter wird mit 2x 300 ml Methanol gewaschen. Die vereinigten methanolischen Lösungen werden im Vakuum bei 70°C Badtemperatur vollständig eingedampft.

Der hochviskose Destillationsrückstand wird am Wasserbad bei 70°C mit 300 ml deionisiertem Wasser versetzt und auf 0°C abgekühlt. Unter Kühlung wird mit 343,8 g 95%iger Schwefelsäure der pH auf 1,5 gestellt, nach einer Nachreaktionszeit wird der dicke Kristallbrei mit 900 ml deionisiertem Wasser versetzt.

Der Kristallbrei wird mit 2 l Aceton über Nacht bei 0°C gerührt. Die Kristalle werden abfiltriert und mit 2x 250 ml eines Gemisches aus Wasser/Aceton im Verhältnis 1:2 und mit 2x 500 ml reinem Aceton gewaschen.

Die vereinigte organische Lösung wird unter Vakuum bei 70°C Badtemperatur vollständig eingedampft, der verbleibende zähe Rückstand wird mit insgesamt 600 ml Eisessig versetzt und durch Zugabe von 5 l Aceton bei Raumtemperatur wird das Produkt ausgefällt. Die Suspension wird auf 5°C gekühlt, das Produkt abfiltriert, mit 550 ml Eisessig/Aceton im Verhältnis 1:10 und 2x 500 ml Aceton gewaschen und bei 20°C unter Vakuum getrocknet.
Ausbeute: 272,1 g

### Beispiel 2

### (Vergleichsbeispiel)

### Herstellung von (S)-(+)-1,2-Diaminopropan-N,N,N',N'-tetraessigsäuremethylester

Die Veresterung wird mit der isolierten (S)-(+)-1,2-Diaminopropan-N,N,N',N'-tetraessigsäure wie folgt durchgeführt:
37,5 g (S)-(+)-1,2-Diaminopropan-N,N,N',N'-tetraessigsäure werden zusammen mit 756 ml Methanol und 22,5 g 95%iger Schwefelsäure 20 h unter Rückfluss erhitzt. Die abgekühlte Lösung wird mit insgesamt 41,5 g Natriumhydrogencarbonat neutralisiert und unter Vakuum bis zur Trockenen eindestilliert. Der verbleibende Rückstand wird zwischen 300 ml deionisiertem Wasser und 300 ml tert-Butylmethylether verteilt und die wässrige Phase mit 2x 150 ml tert-Butylmethylether extrahiert. Die vereinigten organischen Phasen werden mit Natriumsulfat getrocknet, abfiltriert und das Lösungsmittel wird unter Vakuum bis zur Trockenen eingedampft (Rohausbeute: 20,7 g).

Das Rohprodukt wird in 300 ml eines Gemisches aus tert-Butylmethylether/Petrolether -60/95 im Verhältnis 1:2 gelöst, mit 45 g Kieselgel 0,06-0,2 mm 30 min gerührt und abfiltriert. Der Rückstand wird mit 2x 50 ml des vorstehenden LösungsmittelGemisches gewaschen und das Filtrat wird unter Vakuum bis zur Trockenen eingedampft.
Ausbeute: 6,9 g farbloses Öl (Methylester)

### Analysendaten:

| Elementaranalyse: | | C | H | N | O |
|---|---|---|---|---|---|
| C₁₅H₂₆N₂O₈ | ber: | 49,72 | 7,23 | 7,73 | 35,32 |
| | gef: | 49,84 | 7,39 | 7,47 | |
| Drehwert [α]_{D}²⁰ (c=4; Methanol): +3,1° | | | | | |
| 1H-NMR: | 0,97 (d, 3H; -CH-CH₃); 2,49 (, 1H; N-CH-CH₂-); 2,83 (dd, 2H; N-CH-CH₂-); 3,5 (s, 4H; N-CH₂-CO); 3,55 (s, 4H; N-CH₂-CO); 3,61 (s, 12H; COO-CH₃) | | | | |
| 13C-NMR: | 15,0 (q; -CH-CH₃); 51,22 (q; O-CH₃); 51,38 (q; O-CH₃); 52,07 (t; N-CH₂-); 54,99 (t; N-CH₂-); 55,96 (d; N-CH-); 58,08 (t; CH-CH₂-N); 171,69 (s; -CO-); 172,31 (s; -CO-) | | | | |

### Beispiel 3a

### (Vergleichsbeispiel)

### Herstellung von (S)-(+)-1,2-Diaminopropan-N,N,N',N'-tetraessigsäureethylester

Die Veresterung wird mit der isolierten (S)-(+)-1,2-Diaminopropan-N,N,N',N'-tetraessigsäure wie folgt durchgeführt:
25,0 g (S)-(+)-1,2-Diaminopropan-N,N,N',N'-tetraessigsäure werden zusammen mit 725 ml Ethanol und 15,0 g 95%iger Schwefelsäure 120 h unter Rückfluss erhitzt. Die abgekühlte Lösung wird mit insgesamt 27,5 g Natriumhydrogencarbonat neutralisiert und unter Vakuum bis zur Trockene eingedampft. Der verbleibende Rückstand wird zwischen 200 ml deionisiertem Wasser und 200 ml tert-Butylmethylether verteilt und die wässrige Phase wird mit 2x 100 ml tert-Butylmethylether extrahiert. Die vereinigten organischen Phasen werden mit Natriumsulfat getrocknet, abfiltriert und das Lösungsmittel wird unter Vakuum bis zur Trockene abgedampft (Rohausbeute: 19,7 g).

Das Rohprodukt wird in 300 ml Petrolether 60/95 gelöst, mit 40 g Kieselgel 0,06-0,2 mm 30 min gerührt, abfiltriert, der Rückstand wird mit 2x 50 ml Lösungsmittel gewaschen und das Filtrat unter Vakuum bis zur Trockene eingedampft.
Ausbeute: 7,1 g farbloses Öl (Ethylester)

### Analysendaten:

| Elementaranalyse: | | C | H | N | O |
|---|---|---|---|---|---|
| C₁₉H₃₄N₂O₈ | ber: | 54,53 | 8,19 | 6,69 | 30,58 |
| | gef: | 54,51 | 8,36 | 6,56 | |
| Drehwert [α]_{D}²⁰ (c-4; Methanol): +1,1° | | | | | |
| 1H-NMR: | 1,08 (d, 3H; -CH-CH₃); 1,15-1,35 (dd, 12H; -CH₂-CH₃); 2,5 (m, 1H, N-CH-CH₂-); 2,85-3,15 (m, 2H; N-CH-CH₂-); 3,5 (s, 4H; N-CH₂-CO); 3,6 (s, 4H; N-CH₂-CO); 4,0-4,3 (m, 8H; COO-CH₂-CH₃) | | | | |
| 13C-NMR: | 13,96 (q; -CH2-CH₃); 14,0 (q; -CH₂-CH₃); 15,12 (q; -CH-CH₃); 52,27 (t; N-CH₂-CO); 55,28 (t; N-CH₂-CO); 56,0 (d; N-CH-CH₂-); 58,2 (t; CH-CH₂-N); 60,08; 60,15 2x(t; COO-CH₂-); 171,22 (s; CO); 171,87 (s; CO) | | | | |

### Beispiel 3b

### Herstellung von (S)-(+)-1,2-Diaminopropan-N,N,N',N'-tetraessigsäureethylester

50 g (S)-(-)-Diaminopropandihydrochlorid und 192,8 g Chloressigsäure in 321 ml Wasser werden mit 190,4 g Natronlauge in 343 ml Wasser behandelt und während 132 Stunden bei 45°C behandelt. Das Wasser wird abgedampft und die entstehende dicke Aufschlämmung wird mit 100 ml Ethanol versetzt und abermals vollständig abgedampft. Der Rückstand wird in 900 ml Ethanol aufgenommen, mit 90 ml konzentrierter Schwefelsäure behandelt und während 46 Stunden zum Rückfluss erhitzt. Das Reaktionsgemisch wird auf Umgebungstemperatur abgekühlt und die Säure wird durch Zugabe von 240 g Natriumbicarbonat neutralisiert. Der Niederschlag wird abfiltriert, mit 150 ml Ethanol nachgewaschen, das Filtrat wird eingedampft und der ölige Rückstand wird in 250 ml Toluol suspendiert. Nach ausreichender Extraktion mit 2 N Chlorwasserstoffsäure wird die wässrige Phase mit festem Natriumcarbonat (ca. 75 g) neutralisiert und mit etwa 375 ml Toluol extrahiert. Die vollständige Verdampfung der Lösungsmittel liefert 134 g des Ethylesters als schwach gelbes Öl. Eine analytische Probe wurde durch säulen-chromatographische Reinigung über Kieselgel gewonnen.

### Analysendaten:

| Elementaranalyse: | | C | H | N | O |
|---|---|---|---|---|---|
| C₁₉H₃₄N₂O₈ | ber: | 54,53 | 8,19 | 6,69 | 30,58 |
| | gef: | 54,18 | 8,36 | 6,59 | |
| Drehwert | [α]_{D}²⁰ (c=10; Methanol): +8,6° | | | | |
| 1H-NMR: | 1,02 (d, 3H; -CH-CH₃); 1,21-1,27 (dd, 12H; -CH₂-CH₃); 2,5 (m, 1H, N-CH-CH₂-); 2,85-3,07 (m, 2H; N-CH-CH₂-); 3,5 (s, 4H; N-CH₂-CO) ; 3,6 (s, 4H; N-CH₂-CO); 4,05-4,15 (m, 8H; COO-CH₂-CH₃) | | | | |
| 13C-NMR: | 14,27 (q; -CH₂-CH₃); 14,30 (q; -CH₂-CH₃); 15,41 (q; -CH-CH₃);, 52,77 (t; N-CH₂-CO); 55,60 (t; N-CH₂-CO); 56,31 (d; N-CH-CH₂-); 58,51 (t; CH-CH₂-N); 60,44; 60,52 2x(t; COO-CH₂-); 171,56 (s; CO); 172,22 (s; CO) | | | | |

### Beispiel 4a

### (Vergleichsbeispiel)

### Herstellung von (S)-(-)-1,2-Diaminopropan-N,N,N',N'-tetraessigsäureisopropylester

Die Veresterung wird mit der isolierten (S)-(+)-1,2-Diaminopropan-N,N,N',N'-tetraessigsäure wie folgt durchgeführt:
25,0 g (S)-(+)-1,2-Diaminopropan-N,N,N',N'-tetraessigsäure werden zusammen mit 950 ml Isopropanol und 15,0 g 95%iger Schwefelsäure 162 h unter Rückfluss erhitzt. Die abgekühlte Lösung wird mit insgesamt 27,5 g Natriumhydrogencarbonat neutralisiert und unter Vakuum bis zur Trockenen eingedampft. Der verbleibende Rückstand wird zwischen 200 ml deionisiertem Wasser und 200 ml tert-Butylmethylether verteilt und die wässrige Phase wird mit 1x 100 ml tert-Butylmethylether extrahiert. Die vereinigten organischen Phasen werden mit Natriumsulfat getrocknet, abfiltriert und das Lösungsmittel wird unter Vakuum bis zur Trockenen abgedampft (Rohausbeute: 21,2 g).

Das Rohprodukt wird in 300 ml Petrolether 40/65 gelöst, mit 40 g Kieselgel 0,06-0,2 mm 30 min gerührt, abfiltriert, der Rückstand wird mit 2x 50 ml Lösungsmittel gewaschen und das Filtrat unter Vakuum bis zur Trockenen eingedampft.

Ausbeute: 10,8 g leicht gelbliches Öl (Isopropylester) Analysendaten:

| Elementaranalyse: | | C | H | N | O |
|---|---|---|---|---|---|
| C₂₃H₄₂N₂O₈ | ber: | 58,21 | 8,92 | 5,90 | 26,97 |
| | gef: | 58,12 | 9,08 | 5,70 | |
| Drehwert | [α]_{D}²⁰ (c=4; Methanol): -2,6° | | | | |
| 1H-NMR: | 1,05 (d, 3H; -CH-CH₃); 1,15-1,35 (dd, 24H; iPr-CH-(CH₃)₂); 2,5 (m, 1H, N-CH-CH₂-); 2,85-3,15 (m, 2H; N-CH-CH₂-); 3,5 (2s, 2x 4H; N-CH₂-CO); 5,0 (2q, 4H; iPr-CH-(CH₃)₂). | | | | |
| 13C-NMR: | 15,44 (q; -CH₂-CH₃); 21,79 (q; -CH-(CH₃)₂); 21,85 (q; -CH-(CH₃)₂); 52,72 (t; N-CH₂-CO); 55,88 (t; N-CH₂-CO); 56,25 (d; N-CH-CH₂-); 58,53 (t; CH-CH₂-N); 67,77; 67,79 2x(t; COO-CH-); 170,99 (s; CO); 171,67 (s; CO). | | | | |

### Beispiel 4b

### Herstellung von (S)-(+)-1,2-Diaminopropan-N,N,N',N'-tetraessigsäureisopropylester

50 g (S)-(-)-Diaminopropandihydrochlorid und 192,8 g Chloressigsäure in 321 ml Wasser werden mit 190,4 g Natronlauge in 343 ml Wasser behandelt und während 114 Stunden bei 45°C behandelt. Das Wasser wird abgedampft und der entstehende dicke Rückstand mit einer Mischung aus 90 ml konzentrierter Schwefelsäure in 1500 ml 2-Propanol für 41 Stunden zum Rückfluss erhitzt. Das Reaktionsgemisch wird auf Umgebungstemperatur abgekühlt und die Säure wird durch Zugabe von 240 g Natriumbicarbonat neutralisiert. Der Niederschlag wird abfiltriert, mit 150 ml 2-Propanol nachgewaschen, das Filtrat eingedampft und der ölige Rückstand wird in 250 ml Toluol suspendiert. Nach ausreichender Extraktion mit 2 N Chlorwasserstoffsäure wird die wässrige Phase mit festem Natriumcarbonat (ca. 75 g) neutralisiert und mit etwa 375 ml Toluol extrahiert. Die vollständige Verdampfung der Lösungsmittel liefert 41 g des Isopropylesters als schwach gelbes Öl. Eine analytische Probe wurde durch Wiederholung der extraktive Aufarbeitung und anschließender säulenchromato-graphischen Reinigung über Kieselgel gewonnen.

### Analysendaten:

| Elementaranalyse: | | C | H | N | O |
|---|---|---|---|---|---|
| C₂₃H₄₂N₂O₈ | ber: | 58,21 | 8,92 | 5,90 | 26,97 |
| | gef: | 58,09 | 9,06 | 5,88 | |
| Drehwert | [α]_{D}²⁰ (c=10; Methanol): 0,5° | | | | |
| 1H-NMR: | 1,05 (d, 3H; -CH-CH₃); 1,20-1,22 (dd, 24H; iPr-CH-(CH₃)₂); 2,49 (m, 1H, N-CH-CH₂-); 2,90, 3,04 (m, 2H; N-CH-CH₂-); 3,50, 3,53 (2s, 2x 4H; N-CH₂-CO); 4,99 (2q, 4H; iPr-CH-(CH₃)₂). | | | | |
| 13C-NMR: | 15,53 (q; -CH-CH₃); 21,92 (q; -CH-(CH₃)₂); 21,98 (q; -CH-(CH₃)₂); 52,85 (t; N-CH₂-CO); 56,00 (t; N-CH₂-CO); 56,36 (d; N-CH-CH₂-); 58,63 (t; CH-CH₂-N); 67,92; 67,94 2x(t; COO-CH-); 171,10 (s; CO); 171,79 (s; CO). | | | | |

### Beispiel 5 (erfindungsgemäß)

### Herstellung von (S)-(+)-4,4'-(1-Methyl-1,2-ethandiyl)-bis-(2,6-piperazindion) (Dexrazoxan) (I)

### 5.1. Herstellung von (S)-1,2-Diaminopropan-N-N,N,N',N'-tetraessigsäure-tetramethylester (II)

10 kg (S)-(-)-Diaminopropandihydrochlorid und 38,5 kg Chloressigsäure in 65 l Wasser werden mit 38 kg Natronlauge in 69 l Wasser behandelt und während 70 bis 100 Stunden bei 45°C behandelt. Das Wasser wird abgedampft und die entstehende dicke Aufschlämmung wird mit 80 1 Methanol digeriert, filtriert und der Kuchen wird mit Methanol gewaschen. Das Filtrat wird vollständig abgedampft und der Rückstand wird in 180 1 Methanol aufgenommen, mit 18 1 konzentrierter Schwefelsäure behandelt und während 6 Stunden zum Rückfluss erhitzt. Das Reaktionsgemisch wird auf Umgebungstemperatur abgekühlt und die Säure wird durch Zugabe von 20 bis 25 kg Natriumbicarbonat neutralisiert. Der Niederschlag wird abfiltriert, das Filtrat wird eingedampft und der ölige Rückstand wird in 50 1 Ethylacetat gelöst. Nach ausreichender Extraktion mit 2 N Chlorwasserstoffsäure wird die wässrige Phase mit festem Natriumcarbonat neutralisiert und mit etwa 100 l Ethylacetat extrahiert. Die vollständige Verdampfung der Lösungsmittel liefert etwa 13,5 bis 17,3 kg des gewünschten Methylesters, welcher im nächsten Schritt ohne weitere Reinigung verwendet werden kann.

### 5.2. Cyclisierung zum (S)-(+)-4,4'-(1-Methyl-1,2-ethandiyl)-bis-(2,6-piperazindion) (Dexrazoxan) (I)

Zu einer Lösung von 10 kg (S)-(+)-1,2-Diaminopropan-N,N,N',N'-tetraessigsäuremethylester aus dem vorstehenden Beispiel in 34 1 Formamid werden 4,7 kg gasförmiger Ammoniak zugesetzt und das Reaktionsgemisch wird während etwa 12 Stunden bei 40 bis 50°C unter einem Druck von nicht mehr als 5 bar gehalten. Anschließend wird das Reaktionsgemisch langsam auf 150°C erhitzt, der erhaltene Methanol wird während der Erhitzens abdestilliert und das Reaktionsgemisch wird während 10 bis 12 Stunden bei 140 bis 150°C gehalten. Das Lösungsmittel wird abschließend abdestilliert, der ölige Rückstand wird aus Methanol kristallisiert, um 2,9 bis 3,7 kg Dexrazoxan zu ergeben, welches durch Umkristallisation aus 1,4-Dioxan weiter gereinigt werden kann.

## Patentansprüche

1. Verfahren zur Herstellung einer Verbindung der Formel (I) 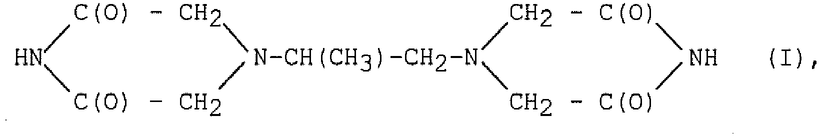 **dadurch gekennzeichnet, dass** es
(a) das Umsetzen von 1,2-Diaminopropan und Chloressigsäure;
(b) das Behandeln des in (a) erhaltenen Reaktionsproduktes ohne vorige Reinigung in einem Alkylalkohol mit starker Säure, vorzugsweise Mineralsäure;
(c) gegebenenfalls die Reinigung der so erhaltenen Verbindung der Formel (II)
(ROOCCH₂)₂N-CHCH₃-CH₂-N(CH₂COOR)₂ (II),
wobei R jeweils für Alkyl steht; und
(d) das Cyclisieren der Verbindung der Formel (II) mit Ammoniak in Formamid umfasst.

2. Verfahren nach Anspruch 1, **dadurch gekennzeichnet, dass** R für (C₁-C₃)Alkyl steht.

3. Verfahren nach Anspruch 1 oder 2, **dadurch gekennzeichnet, dass** die Verbindung der Formel (II) im Schritt (c) von anorganischen Salzen durch Verteilung zwischen einem organischen, mit Wasser unmischbaren Lösungsmittel und Wasser gereinigt wird.

4. Verfahren nach Anspruch 1 oder 2, **dadurch gekennzeichnet, dass** die Verbindung der Formel (II) im Schritt (d) ohne vorherige Isolierung oder Reinigung eingesetzt wird.

5. Verfahren nach einem der Ansprüche 1 bis 4, dadurch gekenzeichnet, dass im Schritt (a) (S)-1,2-Diaminopropan und Chloressigsäure umgesetzt werden.

6. Verfahren nach einem der Ansprüche 1 bis 5 zur Herstellung von (S)-(+)-4,4'-(1-Methyl-1,2-ethandiyl)-bis-(2,6-piperazindion).

## Claims

1. A method for preparing a compound of the formula (I) 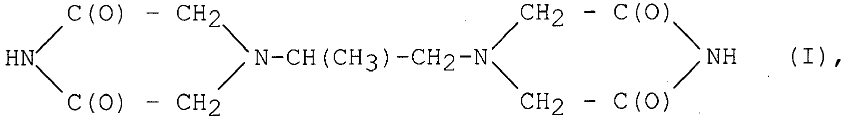 **characterized in that** it comprises
(a) the reaction of 1,2-diaminopropane and chloroacetic acid;
(b) the treatment of the reaction product obtained in step (a) without previous purification with a strong acid, preferably a mineral acid, in an alkylic alcohol;
(c) where appropriate, the purification of the thus obtained compound
(ROOCCH₂)₂N-CHCH₃-CH₂-N(CH₂COOR)₂ (II),
in which R in each case designates alkyl;
(d) the cyclisation of the compound of formula (II) with ammoniac in formamide.

2. A method according to claim 1, **characterized in that** R designates (C₁-C₃)-alkyl.

3. A method according to claim 1 or 2, **characterized in that** in step (c) the compound of formula (II) is purified from inorganic salts by distribution between an organic solvent, which is immiscible with water, and water.

4. A method according to claim 1 or 2, **characterized in that** the compound of formula (II) is used in step (d) without previous separation or purification.

5. A method according to one of the claims 1 to 4, **characterized in that** in step (a) (S)-1,2-diaminopropane is reacted with chloroacetic acid.

6. A method according to one of the claims 1 to 5, for production of (S)-(+)-4,4'-(1-methyl-1,2-ethanediyl)-bis-(2,6-piperazinedione).

## Revendications

1. Un procédé pour la préparation d'un composé de la formule (I) 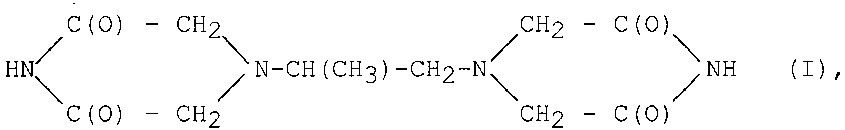 **caractérisé en ce qu'**il comprend
(a) la réaction du 1,2-diaminopropane et de l'acide chloracétique;
(b) le traitement du produit de réaction reçu en (a) sans purification préalable avec un acide fort, de préférence de l'acide minéral, dans un alcool alkylique;
(c) le cas échéant la purification du composé de la formule (II) ainsi reçu
(ROOCCH₂)₂N-CHCH₃-CH₂-N(CH₂COOR)₂ (II),
où R dans chaque cas désigne alkyle; et
(d) la cyclisation du composé de la formule (II) avec de l'ammoniac dans du formamide.

2. Procédé selon la revendication 1, **caractérisé en ce que** R désigne (C₁-C₃)-alkyle.

3. Procédé selon la revendication 1 ou 2, **caractérisé en ce que** le composé de la formule (II) dans l'étape (c) est purifié de sels minéraux par distribution entre un solvant organique qui n'est pas miscible à l'eau et de l'eau.

4. Procédé selon la revendication 1 ou 2, **caractérisé en ce que** le composé de la formule (II) est employé sans séparation ou purification préalable dans l'étape (d).

5. Procédé selon une quelconque des revendications 1 à 4, **caractérisé en ce que** dans l'étape (a) le (S)-1,2-diaminopropane est mis en oeuvre avec l'acide chloracétique.

6. Procédé selon une quelconque des revendications 1 à 5, pour la production du (S)-(+)-4,4'-(1-methyle-1,2-ethanediyle)-bis-(2,6-piperazinedione).
